# FASCICULE DE BREVET EUROPEEN

(11) **EP 3 600 505 B1**
(45) Date de publication et mention de la délivrance du brevet: **07.07.2021**
(21) Numéro de dépôt: 18726517.8
(22) Date de dépôt: 03.04.2018
(51) Int. Cl.: A61M 15/06, A24F 47/00, A61M 11/00, A61M 15/00

(54) **DISPOSITIF PORTABLE D'INHALATION D'AU MOINS UNE COMPOSITION ACTIVE**
TRAGBARE VORRICHTUNG ZUR INHALATION VON MINDESTENS EINER AKTIVEN ZUSAMMENSETZUNG
PORTABLE DEVICE FOR INHALATION OF AT LEAST ONE ACTIVE COMPOSITION

(30) Priorité: 31.03.2017 FR 1752797; 31.03.2017 FR 1752799
(43) Date de publication de la demande: 05.02.2020
(73) Titulaire: ENOVAP, 10430 Rosières-près-Troyes (FR)
(72) Inventeur: SCHECK, Alexandre, 10430 Rosières-près-Troyes (FR); ABULFEDA, Julien, 10430 Rosières-près-Troyes (FR); DELAHAYE, Valentin, 10430 Rosières-près-Troyes (FR); MATTE, Jean-Baptiste, 10430 Rosières-près-Troyes (FR); FISCHER, Alexis, 10430 Rosières-près-Troyes (FR); DE LACROIX DE RAVIGNAN, Aymard, 10430 Rosières-près-Troyes (FR); HARANG, Marie, 10430 Rosières-près-Troyes (FR); MONTANARI, Damien, 10430 Rosières-près-Troyes (FR)
(74) Mandataire: Cornuejols, Georges
(86) Numéro de dépôt international: PCT/FR2018/050828
(87) Numéro de publication internationale: WO 2018/178605

(56) Documents cités:
- WO-A1-95/01137
- WO-A1-97/18846
- WO-A1-2015/150699

## Description

### DOMAINE TECHNIQUE DE L'INVENTION

La présente invention vise un dispositif portable d'inhalation d'au moins une composition active. Elle s'applique, notamment, au domaine des dispositifs médicaux.

### ÉTAT DE LA TECHNIQUE

L'inhalation est une technique d'administration de traitements thérapeutiques mise en oeuvre depuis des millénaires.

L'inhalation permet la délivrance rapide et efficace de substances actives dans le système circulatoire via les poumons. Le principal avantage de cette technique consiste en la rapidité d'action de la substance active inhalée, par rapport à l'administration orale par exemple. Contrairement aux substances administrées oralement, les substances inhalées ne traversent pas le foie, qui détruit de nombreuses molécules d'intérêt.

L'inhalation est aussi la voie de traitement la plus efficace pour administrer des substances actives localement pour le traitement des maladies pulmonaires.

L'inconvénient des inhalateurs actuels est qu'ils ne permettent pas un dosage adaptable de la dose administrée au patient.

Parmi les inhalateurs thérapeutiques proposés sur le marché, une majorité se basent sur les techniques d'aérosolisation par la dépressurisation d'un gaz comprimé contenant la substance active ou par nébulisation de la substance active. Les inhaleurs pressurisés et les nébuliseurs ont un avantage majeur sur les vaporisateurs personnels car les substances actives aérosolisées ne sont pas soumises à une dégradation thermique. Un meilleur contrôle sur le procédé d'aérosolisation des formulations peut être pertinents pour certaines thérapies utilisant des substances actives sensibles à la dégradation thermique.

On connait la demande de brevet internationale WO97/18846 qui divulgue un distributeur de substance active par nébulisation contrôlé de manière similaire au contrôle d'imprimantes à jet d'encre.

### OBJET DE L'INVENTION

L'invention est définie par le jeu de revendications ci-joint.

La présente divulgation vise un dispositif portable d'inhalation d'au moins une composition active, qui comporte :
- au moins un réservoir de stockage d'une composition active,
- au moins une chambre intermédiaire connectée à au moins un réservoir,
- une conduite d'inhalation reliée à la chambre intermédiaire,
- un moyen d'aérosolisation de composition active issue d'un réservoir et
- un régulateur de la quantité de composition active aérosolisée en fonction d'une commande représentative d'une quantité de composition active à aérosoliser reçue par le régulateur.

Grâce à ces dispositions, la quantité de composition active inhalée peut être régulée directement à l'intérieur du dispositif portable. En particulier, la mise en oeuvre de plusieurs réservoirs et, pour chaque réservoir, d'un moyen de transformation de la composition active en un aérosol susceptible d'être inhalé, permet de cumuler les avantages de chaque type d'aérosolisation dans un dispositif unique. Lorsqu'une seule composition active est employée, et que deux réservoirs sont présents, il est possible de doser la quantité de substance active en fonction de l'alimentation de la chambre intermédiaire par au moins un réservoir.

Dans des modes de réalisation, au moins un moyen d'aérosolisation est un nébuliseur de composition active liquide stockée dans un réservoir.

Ces modes de réalisation permettent la formation d'un aérosol sans dégradation thermique de la composition active.

Dans des modes de réalisation, le nébuliseur comporte un tamis présentant des orifices et un moyen de mise en oscillation du tamis, le régulateur comportant un moyen d'obturation d'au moins une partie des orifices en fonction de la quantité de composition active à aérosoliser.

Ces modes de réalisation permettent de contrôler la quantité de composition active nébulisée.

Dans des modes de réalisation, le nébuliseur comporte un moyen de mise en oscillation d'une surface, le régulateur étant configuré pour commander la mise en oscillation de la surface à une fréquence déterminée en fonction de la quantité de composition active à aérosoliser et/ou de caractéristiques de la composition active à nébuliser.

Ces modes de réalisation permettent de contrôler la quantité de composition active nébulisée.

Dans des modes de réalisation, la surface est un tamis présentant des orifices, le régulateur étant configuré pour commander la mise en oscillation du tamis à une fréquence déterminée en fonction de la quantité et/ou de caractéristiques de la composition active à nébuliser.

Ces modes de réalisation permettent de contrôler la quantité de composition active nébulisée.

Dans des modes de réalisation, la surface est localisée en partie basse du réservoir de manière à transmettre des vibrations à la composition active, le régulateur étant configuré pour commander la mise en oscillation à une fréquence déterminée en fonction de la quantité et/ou de caractéristiques de la composition active à nébuliser.

Ces modes de réalisation permettent de contrôler la quantité de composition active nébulisée.

Dans des modes de réalisation, le nébuliseur comporte une pluralité de tamis présentant des porosités différentes et un moyen de mise en oscillation de chaque tamis indépendamment, le régulateur étant configuré pour commander la mise en oscillation d'au moins un des tamis en fonction de la quantité et/ou de caractéristiques de composition active à nébuliser.

Dans des modes de réalisation, le dispositif objet de la présente invention comporte un vaporisateur de composition active liquide stockée dans un réservoir.

Ces modes de réalisation permettent de combiner à la fois une composition active nébulisée et une composition active vaporisée.

Dans des modes de réalisation, le dispositif objet de la présente invention comporte au moins deux réservoirs contenant une composition active à aérosoliser, l'un comportant une composition active contenant une substance active et l'autre comportant une composition active comportant une concentration moindre, ou nulle, de ladite substance active, chaque moyen d'aérosolisation associé à un dit réservoir étant commandé en fonction de la quantité et/ou de caractéristiques de la composition active à aérosoliser.

Dans des modes de réalisation, le dispositif objet de la présente invention comporte un réservoir pressurisé, comportant de l'oxygène ou tout autre gaz pressurisé pouvant contenir une substance active sous forme de solution ou de suspension, relié à la chambre intermédiaire, l'aérosolisation de l'oxygène ou du gaz pressurisé étant commandé par le régulateur.

Ces modes de réalisation permettent à l'utilisateur d'inhaler de l'oxygène ou tout autre gaz pressurisé en plus de la composition active.

Dans des modes de réalisation, au moins deux réservoirs sont pressurisés, l'un comportant une composition active pressurisée contenant une substance active et l'autre comportant une composition active pressurisée comportant une concentration moindre, ou nulle, de ladite substance active, au moins un réservoir étant connecté à la chambre intermédiaire agissant comme chambre d'expansion pour les fluides y étant libérés.

Dans des modes de réalisation, le régulateur est configuré pour commander une durée d'aérosolisation de la composition active issue d'au moins un des deux réservoirs en fonction d'une quantité de substance active, contenue dans la composition, à délivrer.

Ces modes de réalisation permettent de contrôler la quantité de composition active inhalée par l'utilisateur.

Dans des modes de réalisation, le dispositif objet de la présente invention comporte au moins deux réservoirs, chaque réservoir comportant une composition active différente.

Ces modes de réalisation permettent de combiner, en une inhalation, les effets de deux compositions actives.

Dans des modes de réalisation, le dispositif objet de la présente invention comporte au moins deux moyens d'aérosolisation.

Dans des modes de réalisation, au moins deux moyens d'aérosolisation sont associés, chacun, à un réservoir distinct, le dispositif comportant au moins deux réservoirs.

Dans des modes de réalisation, au moins un premier moyen d'aérosolisation est un nébuliseur et au moins un deuxième moyen d'aérosolisation est un nébuliseur.

Dans des modes de réalisation, au moins un premier moyen d'aérosolisation est un moyen de détente d'un contenu pressurisé et au moins un deuxième moyen 'aérosolisation est un moyen de détente d'un contenu pressurisé.

Dans des modes de réalisation, au moins deux moyens d'aérosolisation mettent en oeuvre une technique d'aérosolisation différente.

Dans des modes de réalisation, au moins un premier moyen d'aérosolisation est un nébuliseur et au moins un deuxième moyen d'aérosolisation est un vaporisateur.

Dans des modes de réalisation, au moins un premier moyen d'aérosolisation est un nébuliseur et au moins un deuxième moyen d'aérosolisation est un moyen de détente d'un contenu pressurisé dans un réservoir.

Dans des modes de réalisation, au moins un premier moyen d'aérosolisation est un vaporisateur et au moins un deuxième moyen d'aérosolisation est un moyen de détente d'un contenu pressurisé dans un réservoir.

Dans des modes de réalisation, au moins un vaporisateur est un vaporisateur à coupelle échauffée par conduction ou convection.

Dans des modes de réalisation, au moins un vaporisateur est un vaporisateur à résistance chauffante.

Dans des modes de réalisation, au moins un vaporisateur est un vaporisateur à maillage chauffant.

Dans des modes de réalisation, la chambre intermédiaire stocke un composé chauffé suite à la mise en actionnement d'au moins un vaporisateur de composition active contenue dans un réservoir.

Dans des modes de réalisation, la chambre intermédiaire comporte un élément chauffant le composé stocké dans ladite chambre.

Dans des modes de réalisation, l'élément chauffant est positionné à proximité d'un maillage ou d'une coupelle contenant le composé.

Dans des modes de réalisation, l'élément chauffant est positionné au moins en partie autour et/ou au centre du composé.

Dans des modes de réalisation, la chambre intermédiaire est amovible du dispositif.

### BRÈVE DESCRIPTION DES FIGURES

D'autres avantages, buts et caractéristiques particulières de l'invention ressortiront de la description non limitative qui suit d'au moins un mode de réalisation particulier du dispositif objet de la présente invention, en regard des dessins annexés, dans lesquels :
- la figure 1 représente, schématiquement, un premier mode de réalisation particulier du dispositif objet de la présente invention,
- la figure 2 représente, schématiquement, un deuxième mode de réalisation particulier du dispositif objet de la présente invention,
- la figure 3 représente, schématiquement, un troisième mode de réalisation particulier du dispositif objet de la présente invention,
- la figure 4 représente, schématiquement, un quatrième mode de réalisation particulier du dispositif objet de la présente invention,
- la figure 5 représente, schématiquement, un cinquième mode de réalisation particulier du dispositif objet de la présente invention,
- la figure 6 représente, schématiquement, un sixième mode de réalisation particulier du dispositif objet de la présente invention,
- la figure 7 représente, schématiquement, un septième mode de réalisation particulier du dispositif objet de la présente invention,
- la figure 8 représente, schématiquement, un huitième mode de réalisation particulier du dispositif objet de la présente invention et
- la figure 9 représente, schématiquement, un neuvième mode de réalisation particulier du dispositif objet de la présente invention.

### DESCRIPTION D'EXEMPLES DE RÉALISATION DE L'INVENTION

La présente description est donnée à titre non limitatif, chaque caractéristique d'un mode de réalisation pouvant être combinée à toute autre caractéristique de tout autre mode de réalisation de manière avantageuse.

On note dès à présent que les figures ne sont pas à l'échelle.

Par composition active, on entend toute composition comportant au moins une substance active sous forme solide, liquide ou gazeuse ou toute combinaison de ces états, ladite substance comprenant une molécule active dont l'administration, par voie d'inhalation à un patient, présente un effet thérapeutique. Une telle substance active est, par exemple :
- de la nicotine,
- un opioïde ou
- du THC (pour « Δ-9-tétrahydrocannabinol »).

Lorsque cette substance active présente une forme liquide, elle peut être diluée dans une base contenant par exemple de l'eau saline, du N-acétylcystéine et/ou une solution isotonique, du PG (pour « Propylène Glycol ») ou du VG (pour « Vegetal Glycerine », traduit par glycérine végétale. Des agents aromatiques peuvent être ajoutés à cette base. De même, des vitamines stables dans l'eau ou des extraits de plantes aqueux peuvent être adjoints à cette substance active. Cet ensemble forme alors la composition active. La composition active peut présenter une forme liquide, solide ou gazeuse. Par exemple, la composition active peut présenter une forme d'huile concentrée.

Lorsque cette substance active est contenue dans un gaz comprimé sous forme liquide, elle peut être en solution (solubilisée dans le gaz compressé) ou en suspension (des particules de substance active sont suspendues dans le gaz liquide compressé). Un agent aromatique peut être ajouté à cette base, ainsi que des vitamines stables dans le gaz.

La composition active peut également prendre la forme d'extraits de plantes et/ou de plantes séchées.

Bien que non référencée, chaque mode de réalisation du dispositif, 100, 200, 300 et 400, 500, 600, 700 et 800, comporte une batterie permettant l'alimentation électrique autonome de l'ensemble des composants électriques et électroniques du dispositif.

Comme on le comprend à la lecture de la description ci-dessous, le dispositif objet de la présente invention comporte au moins un réservoir, muni d'un moyen d'aérosolisation de la composition active contenue dans ledit réservoir. Préférentiellement, le dispositif comporte au moins deux réservoirs, chaque réservoir présentant soit une composition active différente, soit un moyen d'aérosolisation différent. Ceci permet de combiner les avantages de deux modes d'aérosolisation différents ou de deux compositions actives différentes au sein d'une seule inhalation et grâce à un dispositif unique.

Par aérosolisation, on entend, par exemple, la nébulisation d'une composition, la vaporisation d'une composition ou la dépressurisation d'une composition pressurisée dans un réservoir.

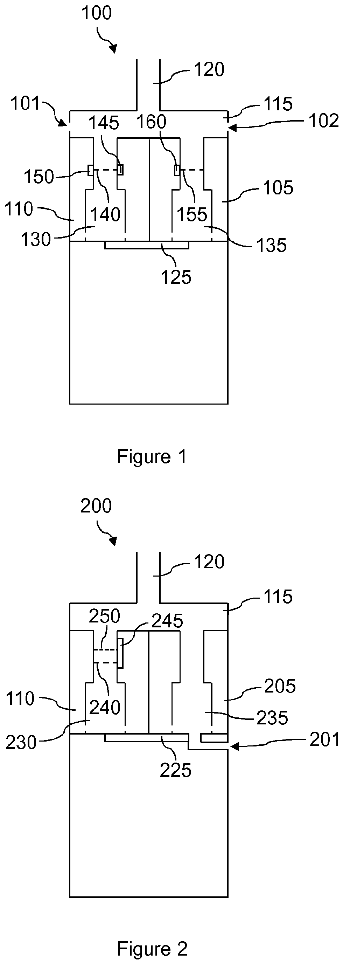

On observe, sur la figure 1, qui n'est pas à l'échelle, une vue schématique d'un mode de réalisation du dispositif 100 objet de la présente invention. Ce dispositif 100 portable d'inhalation d'au moins une composition active, comporte :
- au moins un réservoir, 105 et/ou 110 de stockage d'une composition active,
- au moins une chambre intermédiaire 115 connectée à au moins un réservoir,
- une conduite 120 d'inhalation reliée à au moins une chambre intermédiaire,
- un moyen, 130 et/ou 135, d'aérosolisation de composition active issue d'un réservoir et
- un régulateur 125 de la quantité de composition active aérosolisée en fonction d'une commande représentative d'une quantité de composition active à aérosoliser reçue par le régulateur.

Chaque réservoir, 105 et/ou 110, est, par exemple, un volume étanche muni d'une ouverture reliée à la chambre intermédiaire 115. Dans des variantes, chaque réservoir, 105 et/ou 110, comporte également une entrée d'alimentation en composition active dans laquelle la substance active peut être plus ou moins diluée.

Lorsque le dispositif 100 comporte un nébuliseur de la composition active contenue dans un réservoir, 105 ou 110, l'ouverture de ce réservoir, 105 ou 110, est reliée à un nébuliseur, le nébuliseur étant relié à la chambre intermédiaire 115.

Le dispositif 100 représenté en figure 1 comporte deux réservoirs, 105 et 110. Toutefois, le dispositif 100 peut comporter un seul réservoir, 105 ou 110, ou un nombre de réservoirs supérieur à deux, au moins un réservoir étant relié à la chambre intermédiaire.

La chambre intermédiaire 115 est, par exemple, un volume étanche présentant au moins deux types d'ouvertures : l'un correspondant aux ouvertures reliant la chambre intermédiaire 115 à au moins un réservoir, 105 et/ou 110 et l'autre correspondant à l'ouverture reliant la chambre intermédiaire 115 à la conduite d'inhalation 120.

Lorsque le dispositif 100 comporte un nébuliseur de la composition active sous forme liquide contenue dans un réservoir, 105 et/ou 110, la chambre intermédiaire 115 comporte préférentiellement au moins une ouverture, 101 et/ou 102, agissant comme entrée d'air lors de l'aspiration du patient sur la conduite d'inhalation 120. Ces ouvertures, 101 et 102, peuvent être remplacées par au moins une ouverture au niveau d'au moins un réservoir, lorsqu'un tel réservoir est muni, par exemple, d'un vaporisateur de composition active. Ces entrées d'air, 101 et/ou 102, permettent de provoquer un entrainement de la composition active nébulisée venant d'un réservoir, 105 et/ou 110 vers la conduite d'inhalation 120. La mise en vibration d'au moins une membrane, 140 ou 155, peut être commandée lors de la réception d'une commande par le régulateur 125. Cette commande peut être émise lors de la pression d'un bouton, sur le dispositif 100, par l'utilisateur ou par un terminal tiers, tel un ordiphone par exemple.

Le régulateur 125 est, par exemple, un circuit électronique de commande configuré pour, en fonction de la commande reçue, déclencher l'aérosolisation d'une quantité déterminée de composition active depuis chaque réservoir, 105 et/ou 110, dans la chambre intermédiaire 115. La nature de la commande émise par le régulateur 125 dépend de l'état de la composition active stockée dans chaque réservoir, 105 et/ou 110 et de l'éventuelle transformation de cette composition active réalisée dans le dispositif 100.

La commande reçue par le régulateur 125 peut être reçue via une antenne configurée pour recevoir des signaux électromagnétiques émis selon le standard IEEE 802.11, dit « Wifi », ou selon le standard Bluetooth (Marque déposée) par exemple. Alternativement l'antenne est configurée pour recevoir tout signal sans-fil émis sur une liaison point-à-point ou via un réseau de données de type internet ou réseau de données mobile.

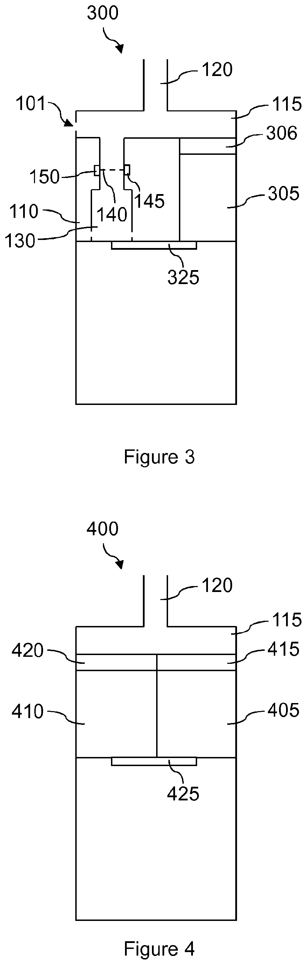

Dans une variante, telle que représentée en figure 3, le dispositif 300 comporte un seul réservoir 305 d'une composition active prenant la forme d'un gaz une fois relâchée, le régulateur 125 ouvrant le passage entre le réservoir 305 et la chambre intermédiaire pour une durée déterminée en fonction de la quantité de composition active à aérosoliser.

Dans une autre variante, telle que représentée en figure 4, le dispositif 400 comporte deux réservoirs, 405 et 410, comportant chacun une composition active contenant différentes concentrations d'une même composition active. En fonction de la quantité de substance active à aérosoliser, le régulateur 125 permet l'aérosolisation d'un volume déterminé de composition active issu de chaque réservoir, 105 et 110, de sorte à ce qu'à volume constant inhalé par l'utilisateur, la quantité de substance active aérosolisée diffère en fonction de l'aérosolisation depuis chaque réservoir.

Dans le mode de réalisation représenté en figure 1, au moins un moyen d'aérosolisation est un nébuliseur 130 de la composition active stockée dans le réservoir 110. Le dispositif 100, tel que représenté en figure 1, comporte également un nébuliseur 135 de la composition active contenue dans le réservoir 105. Dans des variantes, le dispositif 100 comporte seulement l'un de ces nébuliseurs, 130 ou 135.

On rappelle ici qu'un nébuliseur est un appareil permettant de transformer certains liquides en un nuage de particules extrêmement fines, ou brume de particules, et ce, à froid. Pour les nébuliseurs ultrasoniques, la transformation est faite grâce à une surface vibrante actionnée par les ultrasons. Pour les nébuliseurs pneumatiques, la nébulisation est générée par l'introduction d'un gaz dans la composition active à nébuliser.

Dans des variantes, le nébuliseur 130 et 135, comportant au moins une surface, membrane ou tamis, 140 et/ou 155, est mis en oscillation sous la contrainte d'un moyen de mise en oscillation, 145 ou 160, tel un cristal piézo-électrique par exemple. Dans ces variantes, la surface, membrane ou tamis est préférentiellement localisé au-dessus de la composition active.

Le régulateur 125 est alors configuré pour commander la mise en oscillation de la surface à une fréquence déterminée en fonction de la quantité de composition active à aérosoliser.

Dans le cas de ce nébuliseur à tamis, la vibration entrainant l'oscillation de la membrane perforée d'orifices ou du tamis présentant des orifices entraîne, par capillarité à travers la membrane ou le tamis, la formation de gouttelettes de composition active en suspension dans la chambre intermédiaire 115.

Le régulateur 125 est alors configuré pour commander la mise en oscillation du tamis à une fréquence déterminée en fonction de la quantité et/ou de caractéristiques de la composition active à nébuliser.

Dans des variantes, la surface est localisée en partie basse du réservoir de manière à transmettre des vibrations à la composition active, le régulateur 125 étant configuré pour commander la mise en oscillation à une fréquence déterminée en fonction de la quantité et/ou de caractéristiques de la composition active à nébuliser.

Dans ces variantes, au moins un nébuliseur, 130 et/ou 135, comporte par exemple une coupelle non poreuse contenant un volume de composition active à nébuliser relié à un réservoir, 105 et/ou 110. Le moyen de mise en vibration 145 et/ou 160, qui peut consister en un métal piézo-électrique pouvant être localisé en partie basse du réservoir, provoque la formation de jets en surface de la composition active, ces jets entraînant la formation de gouttelettes demeurant au-dessus de la surface de la composition active. Le moyen de mise en vibration 145 et/ou 160 qui peut être localisé en partie basse du réservoir transmet les vibrations à la composition active à nébuliser, soit directement soit par l'intermédiaire d'un couplage liquidien.

Dans ce dernier cas, la composition active est déposée dans une coupelle hémisphérique en partie immergée dans un réservoir d'eau placé au-dessus du cristal : l'eau sert de liquide de transmission.

Lorsqu'un nébuliseur est employé, la composition active prend la forme d'une composition liquide. Cette composition peut être initialement liquide ou résulter de la fonte d'une composition initialement solide par exemple.

Lorsque le dispositif 100 comporte au moins deux nébuliseurs, 130 et 135, chaque réservoir, 105 et 110, associé stocke une composition présentant une concentration de substance active différente. Ainsi, la régulation de la quantité de substance active inhalée par le patient est réalisée par la nébulisation d'un volume différent de liquide issu de chaque réservoir, 105 et 110, de sorte à ce qu'à volume nébulisé total constant, la quantité de substance active varie en fonction de la proportion du volume issu de chaque réservoir, 105 et 110.

Dans un premier exemple, la composition du premier réservoir comporte 80% en concentration d'une substance active et le deuxième réservoir comporte 20% en concentration de la substance active. Afin d'administrer un volume de solution nébulisée présentant une concentration de 50%, le régulateur 125 commande la nébulisation d'un volume identique issu de chaque réservoir. Pour obtenir un volume de solution nébulisée présentant une concentration de 60%, le régulateur 125 commande la nébulisation d'un volume issu du premier réservoir égal au double du volume issu du deuxième réservoir.

Dans un deuxième exemple, la composition du premier réservoir comporte 80% en concentration d'une substance active et le deuxième réservoir comporte 0% en concentration de la substance active. Afin d'administrer un volume de solution nébulisée présentant une concentration de 40%, le régulateur 125 commande la nébulisation d'un volume identique issu de chaque réservoir. Pour obtenir un volume de solution nébulisée présentant une concentration de 60%, le régulateur 125 commande la nébulisation d'un volume issu du premier réservoir égal au triple du volume issu du deuxième réservoir.

Dans des modes de réalisation, tel que celui représenté en figure 1, un nébuliseur 130 comporte un tamis 140 présentant des orifices et un moyen 145 de mise en oscillation du tamis, le régulateur 125 comportant un moyen 150 d'obturation d'au moins une partie des orifices en fonction de la quantité de composition active à aérosoliser.

Cette obturation est réalisée, par exemple, par une obturation de valves formant tout ou partie du tamis. Ces valves, sensibles à un courant électrique les traversant, s'ouvrent ou se ferment en fonction de la valeur d'intensité ou de tension du courant.

Cette obturation permet de limiter le débit de brume de gouttelettes formée et ainsi la quantité de composition active nébulisée inhalée par le patient en relation à l'air aspiré issue de l'entrée d'air 101 et/ou à la quantité de composition active nébulisée depuis au moins un autre réservoir 105 si un tel autre réservoir 105 est présent.

Dans des variantes, chaque nébuliseur, 130 et 135, présente un fonctionnement identique au nébuliseur 130 tel que décrit ci-dessus.

Dans des modes de réalisation, tel que celui représenté en figure 1, un nébuliseur 135 comporte un tamis 155 présentant des orifices et un moyen 160 de mise en oscillation du tamis, le régulateur 125 étant configuré pour commander la mise en oscillation du tamis à une fréquence déterminée en fonction de la quantité et/ou caractéristiques de composition active à aérosoliser.

La quantité et/ou caractéristiques de composition active nébulisée dépend de la fréquence d'oscillation du tamis 155. Ainsi, cette variation de fréquence permet d'ajuster le débit/et ou caractéristiques de brume de gouttelettes formée et ainsi la quantité et/ou caractéristiques de composition active nébulisée inhalée par le patient en relation à l'air aspiré issu de l'entrée d'air 101 et/ou à la quantité et/ou caractéristiques de composition active nébulisée depuis au moins un autre réservoir 110 si un tel autre réservoir 110 est présent.

Dans des modes de réalisation, tel que celui représenté en figure 1, un nébuliseur 135 comporte un moyen 160 de mise en oscillation, le régulateur 125 étant configuré pour commander la mise en oscillation à une fréquence déterminée en fonction de la quantité et/ou caractéristiques de composition active à aérosoliser.

La quantité et/ou caractéristiques de composition active nébulisée dépend de la fréquence d'oscillation. Ainsi, cette variation de fréquence permet d'ajuster le débit et/ou caractéristiques de brume de gouttelettes formé et ainsi la quantité et/ou caractéristiques de composition active nébulisée inhalée par le patient en relation à l'air aspiré issu de l'entrée d'air 101 et/ou à la quantité et/ou caractéristiques de composition active nébulisée depuis au moins un autre réservoir 110 si un tel autre réservoir 110 est présent.

Dans des variantes, chaque nébuliseur, 130 et 135, présente un fonctionnement identique au nébuliseur 135 tel que décrit ci-dessus.

Ainsi, comme on le comprend, la régulation de la quantité de composition active déterminée peut être réalisée :
- lorsque le dispositif 100 comporte plusieurs réservoirs, par régulation du rapport de débits de composition active nébulisée par chaque réservoir et
- lorsque le dispositif 100 comporte plusieurs réservoirs, par régulation du rapport du débit de composition active nébulisée dans un réservoir par rapport à un débit d'une autre aérosolisation de la composition active, tel décrit en regard des figures 2 ou 3 par exemple.

On observe, sur la figure 2, qui n'est pas à l'échelle, une vue schématique d'un mode de réalisation du dispositif 200 objet de la présente invention. Ce dispositif 200 portable d'inhalation d'au moins une composition active, comporte :
- au moins un réservoir, 205 et/ou 110 de stockage d'une composition active,
- au moins une chambre 115 intermédiaire connectée à au moins un réservoir,
- une conduite 120 d'inhalation reliée à au moins une chambre intermédiaire,
- un moyen, 240 et/ou 235, d'aérosolisation de composition active issue d'un réservoir et
- un régulateur 225 de la quantité de composition active aérosolisée en fonction d'une commande représentative d'une quantité de composition active à aérosoliser reçue par le régulateur.

Le réservoir 110, la chambre intermédiaire 115 et la conduite d'inhalation 120 sont, identiques au réservoir 110, à la chambre intermédiaire 115 et à la conduite d'inhalation tels que décrits en regard de la figure 1.

Ce mode de réalisation comporte un nébuliseur 230 comportant une pluralité de membranes, ou tamis, 240 et 250, munies d'orifices permettant le passage de la composition active contenue dans le réservoir 110. Ces tamis, 240 et 250, peuvent être disposés parallèlement le long d'une conduite reliant le réservoir 110 à la chambre intermédiaire 115. Chaque tamis, 240 et 250, présente une porosité différente. Cette porosité peut dépendre du nombre d'orifices présents sur chaque tamis, 240 et 250. Alternativement, les orifices de ces tamis, 240 et 250, présentent, pour chaque tamis, 240 et 250, des dimensions différentes, en longueur, en hauteur et/ou en largeur. Cette différence de dimension provoque, lors de l'oscillation de l'une ou l'autre de ces membranes, la nébulisation de la composition active selon un débit et/ou des caractéristiques d'aérosol variant en fonction de la porosité dudit tamis. De cette manière, les caractéristiques de la brume nébulisées peuvent être contrôlées autant que le rapport de ce débit au débit d'aspiration d'air par une entrée (non représentée) dans la chambre intermédiaire 115 et/ou au débit de nébulisation d'un autre nébuliseur, 130 et/ou 135, ou d'un autre moyen d'aérosolisation de la composition active.

Le dispositif 200 comporte également un moyen 245 de mise en oscillation de chaque tamis indépendamment, le régulateur 225 étant configuré pour commander la mise en oscillation d'au moins un des tamis en fonction de la quantité et/ou des caractéristiques de composition active à aérosoliser. Ce moyen 245 de mise en oscillation présente un fonctionnement similaire au moyen 145 de mise en oscillation tel que décrit en regard de la figure 1. Si chaque tamis est utilisé de façon indépendante, les gouttelettes de brume ne traversent pas nécessairement les deux tamis. Un des tamis peut être déplacé de manière à ne pas rentrer en contact avec la brume produite par l'autre tamis.

Ce dispositif 200 comporte, de manière optionnelle, un deuxième réservoir 205 muni d'un vaporisateur 235 de composition active contenue dans ledit réservoir 205. Ce deuxième réservoir 205 comporte une entrée d'air 201 remplaçant l'entrée d'air, telle que décrite ci-dessus, dans la chambre intermédiaire 115.

Le vaporisateur 235 est, par exemple, une résistante chauffante configurée pour échauffer la composition active et, en fonction de la durée et de la température de chauffe, provoquer un débit et/ou des caractéristiques de vaporisation déterminé. Cette durée et/ou cette température de chauffe sont commandées par le régulateur 225. La composition active à chauffer est initialement liquide.

Dans des variantes, le vaporisateur 235 est, par exemple, une coupe chauffée par conduction ou convection. Dans ces variantes, la composition active est par exemple initialement issue de plantes ou dans un concentré huileux ou d'une forme de cire. Une telle huile ou cire représente par exemple un concentré issu de plantes.

Dans des variantes, le vaporisateur 235 est, par exemple, un maillage chauffé par conduction ou convection. Dans ces variantes, la composition active est par exemple initialement huileuse, telle des huiles essentielles ou de l'huile de THC par exemple.

Le régulateur 225 détermine et commande, par exemple, un rapport de débits ou de quantité de nébulisation/de vaporisation du liquide contenu dans chaque réservoir, 110 et 205. Ce débit, ou cette quantité, est éventuellement asservi à un débit capté d'inhalation par le patient ou à une commande reçue par le régulateur 225.

Structurellement, le régulateur 225 est, par exemple, identique au régulateur 125.

On observe, sur la figure 3, qui n'est pas à l'échelle, une vue schématique d'un mode de réalisation du dispositif 300 objet de la présente invention. Ce dispositif 300 portable d'inhalation d'au moins une composition active, comporte :
- au moins un réservoir, 305 et/ou 110 de stockage d'une composition active,
- au moins une chambre 115 intermédiaire connectée à au moins un réservoir,
- une conduite 120 d'inhalation reliée à au moins une chambre intermédiaire,
- un moyen, 130 et/ou 135, d'aérosolisation de composition active issue d'un réservoir et
- un régulateur 325 de la quantité de composition active aérosolisée en fonction d'une commande représentative d'une quantité de composition active à aérosoliser reçue par le régulateur.

Le réservoir 110, la chambre intermédiaire 115 et la conduite d'inhalation 120 sont, identiques au réservoir 110, à la chambre intermédiaire 115 et à la conduite d'inhalation tels que décrits en regard de la figure 1.

Le dispositif 300 comporte, de plus, un nébuliseur 130 similaire au nébuliseur 130 tel que décrit en regard de la figure 1. Dans des variantes, ce nébuliseur 130 est remplacé par le nébuliseur 135 tel que décrit en regard de la figure 1 ou le nébuliseur 230 tel que décrit en regard de la figure 2.

Ce dispositif 300 comporte, de plus, un réservoir 305 d'oxygène pressurisé, relié à la chambre 115 intermédiaire, l'aérosolisation d'oxygène étant commandée par le régulateur 325. Ce réservoir d'oxygène 305 peut être remplacé par tout réservoir de gaz pressurisé pouvant contenir une composition active ou non.

L'aérosolisation d'oxygène peut être réalisée via un piston ou via l'ouverture d'une valve.

Le régulateur 325 détermine et commande, par exemple, un rapport du débit ou de quantité de nébulisation du liquide contenu dans le réservoir 110 et du débit ou de quantité d'aérosol de gaz pressurisé issu du réservoir 305. Ce débit, ou cette quantité, est éventuellement asservi à un débit capté d'inhalation par le patient ou à une commande reçue par le régulateur 325.

Structurellement, le régulateur 325 est, par exemple, identique au régulateur 125.

On observe, sur la figure 4, qui n'est pas à l'échelle, une vue schématique d'un mode de réalisation du dispositif 400 objet de la présente invention. Ce dispositif 400 portable d'inhalation d'au moins une composition active, comporte :
- au moins un réservoir, 405 et/ou 410 de stockage d'une composition active,
- au moins une chambre 115 intermédiaire connectée à au moins un réservoir,
- une conduite 120 d'inhalation reliée à au moins une chambre intermédiaire,
- un moyen, 415 et/ou 420, d'aérosolisation de composition active issue d'un réservoir et
- un régulateur 425 de la quantité de composition active aérosolisée en fonction d'une commande représentative d'une quantité de composition active à aérosoliser reçue par le régulateur.

La chambre intermédiaire 115 et la conduite d'inhalation 120 sont, identiques à la chambre intermédiaire 115 et à la conduite d'inhalation telles que décrites en regard de la figure 1.

Dans ce dispositif 400, au moins deux réservoirs 405, 410 sont pressurisés, l'un comportant une composition active pressurisée contenant une substance active et l'autre comportant une composition active pressurisée comportant une concentration moindre, ou nulle, de ladite substance active, au moins un réservoir étant connecté à au moins une chambre intermédiaire 115 agissant comme chambre d'expansion pour les fluides y entrant.

Le régulateur 425 détermine et commande, par exemple, un rapport de débits ou de durées d'aérosolisation du fluide contenu dans les réservoirs, 405 et 410. Le régulateur 425 est configuré pour commander un début et/ou une durée d'aérosolisation depuis au moins un des deux réservoirs, 405 et 410, en fonction de la quantité de substance active à aérosoliser.

Structurellement, le régulateur 425 est, par exemple, identique au régulateur 125.

Dans des variantes, le dispositif, 100, 200, 300 et/ou 400, comporte au moins deux réservoirs, 110, 105, 205, 305, 405 et 410, chaque réservoir comportant une composition active différente.

Par exemple, dans un premier réservoir se trouve une composition active primaire comportant une première substance active à réguler ainsi qu'une deuxième substance active et dans un deuxième réservoir se trouve uniquement une composition active comportant la deuxième substance active, ce qui permet d'administrer la substance active primaire avec une concentration précise et d'administrer en continue la substance active secondaire.

Ainsi, comme on le comprend à la lecture de la description qui précède, le contrôle de la quantité de composition active aérosolisée peut être réalisé au niveau d'un terminal communicant, tel un ordiphone ou un ordinateur personnel par exemple. Ce contrôle spontané peut être intégré à un programme visant à réguler dans le temps la quantité de composition active inhalée. Dans ces variantes, préalablement à chaque inhalation, le terminal communicant transmet au régulateur du dispositif la quantité à libérer pour inhalation par le patient.

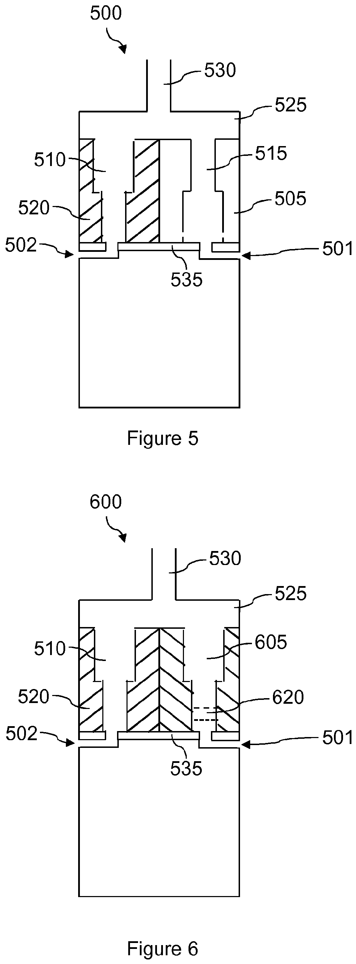

On observe, sur la figure 5, qui n'est pas à l'échelle, une vue schématique d'un mode de réalisation du dispositif objet de la présente invention. Ce dispositif 500 portable de vaporisation d'au moins une composition active, comporte :
- au moins un réservoir, 505 et/ou 510, de stockage d'une composition active,
- pour au moins un réservoir, au moins un vaporisateur, 515 et/ou 520 de la composition active contenue dans ledit réservoir,
- au moins une chambre 525 intermédiaire connectée à au moins un dit réservoir pouvant permettre le mélange des compositions actives vaporisées,
- une conduite 530 d'inhalation reliée à au moins une chambre intermédiaire et
- un actionneur 535 d'au moins un vaporisateur en fonction d'une commande reçue par l'actionneur.

Chaque réservoir, 505 et/ou 510, est, par exemple, un volume étanche muni d'une ouverture reliée à la chambre intermédiaire 515. Dans des variantes, chaque réservoir, 505 et/ou 510, comporte également une entrée d'alimentation en composition active et/ou une entrée d'air, 501 et/ou 502. Chaque entrée d'air, 501 et/ou 502, permet le passage d'air lorsque le patient aspire sur la conduite d'inhalation 530.

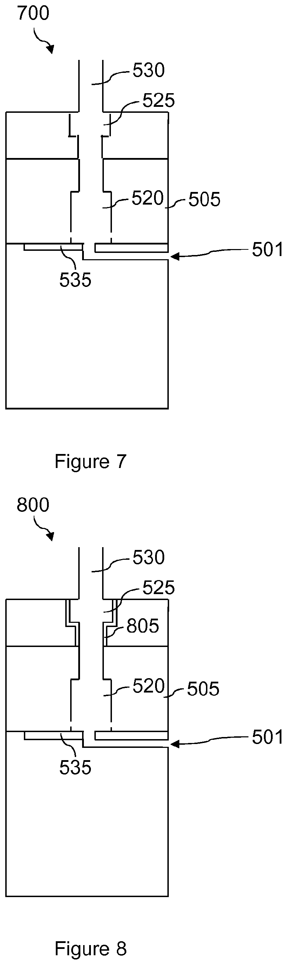

Le dispositif 500 représenté en figure 5 comporte deux réservoirs, 505 et 510. Toutefois, le dispositif 500 peut comporter un seul réservoir, 505 ou 510, ou un nombre de réservoirs supérieur à deux, au moins un réservoir étant relié à la chambre intermédiaire 525. Un tel exemple de dispositif à réservoir unique est visible en figures 7 et 8.

La chambre intermédiaire 525 est, par exemple, un volume étanche présentant au moins deux types d'ouvertures : l'un correspondant aux ouvertures reliant la chambre intermédiaire 525 à au moins un réservoir, 505 et/ou 510 et l'autre correspondant à l'ouverture reliant la chambre intermédiaire 525 à la conduite d'inhalation 530.

Pour chaque réservoir, 505 et 510, le dispositif 500 comporte un vaporisateur, 515 et 520. Chaque vaporisateur, 515 ou 520, est configuré pour provoquer l'évaporation des agents aromatiques et de la composition active chauffée contenue dans le réservoir, 505 ou 510, associé audit vaporisateur, 515 ou 520.

Dans des modes de réalisation, au moins un vaporisateur 520 est un vaporisateur à coupelle échauffée par conduction ou convection.

Lorsque la coupelle est échauffée par conduction, un moyen de chauffe fait monter en température les parois d'un réservoir dans lequel se trouve des extraits de plantes et/ou des plantes séchées ou de la cire et ou une huile concentrée contenant la substance active. Ces extraits de plantes et/ou plantes séchées, huile ou cire sont donc chauffés par contact avec le réservoir. Lorsque l'utilisateur aspire dans le dispositif, l'air traverse les extraits de plantes et/ou des plantes séchées, l'huile ou la cire qui a été chauffé à une certaine température et emporte la ou les substances actives et/ou arômes dans les voies respiratoires de l'utilisateur.

Lorsque la coupelle est échauffée par convection, un moyen de chauffe situé avant le réservoir contenant des extraits de plantes et/ou des plantes séchées, de l'huile concentrée ou de la cire chauffe pour atteindre une température prédéfinie. Une fois celle-ci atteinte l'utilisateur est invité à inhaler ce qui entraine l'arrivée d'un flux d'air qui est chauffé instantanément par le moyen de chauffe avant de passer à travers les extraits de plantes et/ou plantes séchées, la cire, l'huile concentrée ou la cire dans le réservoir. L'air étant chaud cela permet de libérer la ou les substances actives et/ou arômes afin que l'utilisateur les inhale. On appelle cela de la convection car les extraits de plantes et/ou plantes séchées, huile ou la cire sont chauffés par le moyen de chauffe mais indirectement car c'est l'air qui conduit la chaleur

Dans des modes de réalisation, au moins un vaporisateur 515 est un vaporisateur à résistance chauffante.

Dans des modes de réalisation, au moins un vaporisateur est un vaporisateur à maillage chauffant, tel que représenté en figure 6. Le maillage est, par exemple, formé de coton, de céramique ou de métal. Un gel comportant une substance active ou de l'huile essentiel est positionné sur ce maillage ou imbibe ce maillage.

Lorsque le dispositif 500 comporte au moins deux réservoirs, 505 et 510, le dispositif 500 peut comporter n'importe quelle combinaison de vaporisateurs, 515 et 520, associés à chaque réservoir.

L'association de deux types de vaporisateur permet de combiner les effets de ces types de vaporisateurs lors d'une inhalation unique.

Par exemple, un premier réservoir est muni d'un vaporisateur à coupelle ou à maillage chauffant et le contenu du premier réservoir est, par exemple, formé d'extraits de plantes et/ou de plantes séchées et/ou d'une substance active dans un gel et/ou un concentré de substance active et/ou d'agent aromatiques et/ou d'huiles essentielles. Dans des variantes, le contenu du premier réservoir comporte des feuilles séchées de tabac ou du cannabis et/ou de la cire issue de plantes comme par exemple la cire de THC.

Un deuxième réservoir est muni d'un vaporisateur à résistance et contient une base d'aérosol comportant ou non un agent aromatique et/ou une substance active. Cette base d'aérosol est, par exemple, de l'eau, du PG (pour « Propylène Glycol ») ou du VG (pour « Vegetal Glycerine », traduit par glycérine végétale, et/ou une solution isotonique.

L'avantage d'un dispositif tel que décrit en regard de la figure 5 associant chauffe d'extraits de plantes et/ou de plantes séchées, d'huile ou de cire et vaporisation d'une base diluante est que ce dispositif permet d'associer la production d'une vapeur à but récréatif ou thérapeutique ainsi que la vaporisation d'arômes naturels et de compositions actives.

On observe, sur la figure 6, qui n'est pas à l'échelle, une vue schématique d'un mode de réalisation du dispositif objet de la présente invention. Ce dispositif 600 portable de vaporisation d'au moins une composition active, comporte :
- au moins un réservoir, 605 et/ou 510, de stockage d'une composition active,
- pour au moins un réservoir, au moins un vaporisateur, 520 et/ou 620 de la composition active contenue dans ledit réservoir,
- au moins une chambre 525 intermédiaire connectée à au moins un dit réservoir pouvant permettre le mélange des compositions actives vaporisées,
- une conduite 530 d'inhalation reliée à au moins une chambre intermédiaire et
- un actionneur 535 d'au moins un vaporisateur en fonction d'une commande reçue par l'actionneur.

Cette figure 6 est une variante de la figure 5 dans laquelle l'un des vaporisateurs 620 est un vaporisateur à maillage chauffant.

L'avantage d'associer deux systèmes chauffant chacun un extrait de plantes et/ou des plantes séchées, de l'huile ou de la cire est d'offrir plus de flexibilité dans l'utilisation du dispositif. Par exemple, ce dispositif permet de sélectionner une température de chauffe optimale pour chacun des éléments.

On observe, sur la figure 7, qui n'est pas à l'échelle, une vue schématique d'un mode de réalisation du dispositif objet de la présente invention. Ce dispositif 700 portable de vaporisation d'au moins une composition active, comporte :
- au moins un réservoir 505 de stockage d'une composition active,
- pour au moins un réservoir, au moins un vaporisateur 520 de la composition active contenue dans ledit réservoir,
- au moins une chambre 525 intermédiaire connectée à au moins un dit réservoir pouvant permettre le mélange des compositions actives vaporisées,
- une conduite 530 d'inhalation reliée à au moins une chambre intermédiaire et
- un actionneur 535 d'au moins un vaporisateur en fonction d'une commande reçue par l'actionneur.

Dans ce mode de réalisation, la chambre intermédiaire 525 stocke un composé chauffé suite à la mise en actionnement d'au moins un vaporisateur 520 de composition active contenue dans un réservoir 505. L'échauffement peut résulter de la mise en contact du composé avec les vapeurs chaudes et/ou par la proximité du composé avec un élément de chauffe du vaporisateur 520.

On observe, sur la figure 8, qui n'est pas à l'échelle, une vue schématique d'un mode de réalisation du dispositif objet de la présente invention. Ce dispositif 800 portable de vaporisation d'au moins une composition active, comporte :
- au moins un réservoir 505 de stockage d'une composition active,
- pour au moins un réservoir, au moins un vaporisateur 520 de la composition active contenue dans ledit réservoir,
- au moins une chambre 525 intermédiaire connectée à au moins un dit réservoir pouvant permettre le mélange des compositions actives vaporisées,
- une conduite 530 d'inhalation reliée à au moins une chambre intermédiaire et
- un actionneur 535 d'au moins un vaporisateur en fonction d'une commande reçue par l'actionneur.

Dans ce mode de réalisation, la chambre 525 intermédiaire comporte un élément 805 chauffant le composé stocké dans ladite chambre. Cet élément 805 chauffant est, par exemple, une résistance électrique. Dans le mode de réalisation représenté en figure 8, l'élément 805 chauffant est positionné à proximité d'un maillage ou d'une coupelle contenant le composé. Dans des variantes, l'élément 805 chauffant est positionné au moins en partie autour et/ou au centre du composé.

La chambre 525 intermédiaire telle que décrite en regard des figures 7 et 8 est préférentiellement amovible du dispositif 700 et 800.

Les dispositifs décrits en regard des figures 7 et 8 permettent d'associer une production de vapeur, à but thérapeutique ou récréatif, à la délivrance de substances actives et aromatiques. La vapeur en passant par la matière à chauffer sera elle-même infusée par les arômes et substances actives qu'elle permet de libérer.

Le dispositif décrit en regarde de la figure 8 permet d'obtenir une vapeur plus chaude et d'extraire plus facilement la substance active que dans le cas de la figure 7.

Ainsi, comme on le comprend, le dispositif objet de la présente invention peut comporter au moins deux moyens d'aérosolisation distincts. Ces deux moyens d'aérosolisation peuvent être associés, chacun, à un réservoir distinct, le dispositif comportant alors au moins deux réservoirs. Les deux moyens d'aérosolisation peuvent être du même type ou de types différents.

Dans des variantes, telle que celle représentée en figure 1, au moins un premier moyen 130 d'aérosolisation est un nébuliseur et au moins un deuxième moyen 135 d'aérosolisation est un nébuliseur.

Dans des variantes, telle que celle représentée en figure 4, au moins un premier moyen 415 d'aérosolisation est un moyen de détente d'un contenu pressurisé et au moins un deuxième moyen 420 d'aérosolisation est un moyen de détente d'un contenu pressurisé. Un moyen tel de détente correspond, par exemple, correspond par exemple à une ouverture dans un réservoir commandée par un régulateur tel que décrit en regard de la figure 4.

Dans des variantes, au moins deux moyens d'aérosolisation mettent en oeuvre une technique d'aérosolisation différente.

Dans des variantes, telle que celle représentée en figure 2, au moins un premier moyen 230 d'aérosolisation est un nébuliseur et au moins un deuxième moyen 235 d'aérosolisation est un vaporisateur.

Dans des variantes, telle que celle représentée en figure 3, au moins un premier moyen 130 d'aérosolisation est un nébuliseur et au moins un deuxième moyen 306 d'aérosolisation est un moyen de détente d'un contenu pressurisé dans un réservoir.

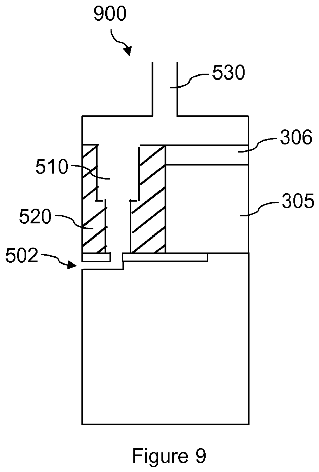

Dans des variantes, telle que celle représentée en figure 9, au moins un premier moyen 520 d'aérosolisation est un vaporisateur et au moins un deuxième moyen 306 d'aérosolisation est un moyen de détente d'un contenu pressurisé dans un réservoir. Un tel vaporisateur 520 est décrit, par exemple, en regard de la figure 5 tandis qu'un tel moyen de détente 306 est décrit en regard de la figure 3. Un moyen tel de détente correspond, par exemple, correspond par exemple à une ouverture dans un réservoir commandée par un régulateur tel que décrit en regard de la figure 3.

## Revendications

1. Dispositif (100, 200, 300, 400, 500, 600, 700, 800) portable d'inhalation d'au moins une composition active, **caractérisé en ce qu'**il comporte :
- au moins deux réservoirs (105, 110, 205, 305, 405, 410, 505, 510, 605) de stockage d'une composition active,
- au moins une chambre (115, 525) intermédiaire connectée à au moins un réservoir,
- une conduite (120, 530) d'inhalation reliée à la chambre intermédiaire,
- au moins deux moyens (130, 135) d'aérosolisation et dans lequel au moins deux moyens (130, 135) d'aérosolisation sont associés, chacun, à un réservoir distinct, et :
- au moins un premier moyen (415) d'aérosolisation est un moyen de détente d'un contenu pressurisé et au moins un deuxième moyen (420) d'aérosolisation est un moyen de détente d'un contenu pressurisé ou
- au moins un premier moyen (230) d'aérosolisation est un nébuliseur et au moins un deuxième moyen (235) d'aérosolisation est un vaporisateur ou
- au moins un premier moyen (130) d'aérosolisation est un nébuliseur et au moins un deuxième moyen (306) d'aérosolisation est un moyen de détente d'un contenu pressurisé dans un réservoir ou
- au moins un premier moyen (520) d'aérosolisation est un vaporisateur et au moins un deuxième moyen (306) d'aérosolisation est un moyen de détente d'un contenu pressurisé dans un réservoir.
- un régulateur (125, 225, 325, 425, 535) de la quantité de composition active aérosolisée en fonction d'une commande représentative d'une quantité de composition active à aérosoliser reçue par le régulateur.

2. Dispositif (100, 200, 300) selon la revendication 1, dans lequel au moins un moyen d'aérosolisation est un nébuliseur (130, 135, 230) de composition active liquide stockée dans un réservoir et dans lequel le nébuliseur (130) comporte :
- un tamis (140) présentant des orifices et un moyen (145) de mise en oscillation du tamis, le régulateur (125) comportant un moyen (150) d'obturation d'au moins une partie des orifices en fonction de la quantité de composition active à aérosoliser ou le régulateur étant configuré pour commander la mise en oscillation du tamis à une fréquence déterminée en fonction de la quantité et/ou de caractéristiques de la composition active à nébuliser ou
- un moyen (160) de mise en oscillation d'une surface, le régulateur (125) étant configuré pour commander la mise en oscillation de la surface à une fréquence déterminée en fonction de la quantité de composition active à aérosoliser et/ou de caractéristiques de la composition active à nébuliser ou
- une pluralité de tamis (240, 250) présentant des porosités différentes et un moyen (245) de mise en oscillation de chaque tamis indépendamment, le régulateur (225) étant configuré pour commander la mise en oscillation d'au moins un des tamis en fonction de la quantité de composition active à aérosoliser.

3. Dispositif (200) selon l'une des revendications 1 à 2, qui comporte un vaporisateur (235) de composition active liquide stockée dans un réservoir (205).

4. Dispositif (100) selon l'une des revendications 1 à 3, dans lequel un réservoir comporte=une composition active contenant une substance active et l'autre réservoir comporte une composition active comportant une concentration moindre, ou nulle, de ladite substance active, chaque moyen d'aérosolisation associé à un dit réservoir étant commandé en fonction de la quantité et/ou de caractéristiques de la composition active à aérosoliser.

5. Dispositif (300) selon l'une des revendications 1 à 4, dans lequel au moins un réservoir (305) est pressurisé, et comporte de l'oxygène ou tout autre gaz pressurisé pouvant contenir une substance active sous forme de solution ou de suspension, relié à la chambre (115) intermédiaire, l'aérosolisation de l'oxygène ou du gaz pressurisé étant commandé par le régulateur (325), au moins un réservoir étant connecté à la chambre intermédiaire (115) agissant comme chambre d'expansion pour les fluides y étant libérés.

6. Dispositif (400) selon la revendication 4, dans lequel le régulateur (425) est configuré pour commander une durée d'aérosolisation de la composition active contenue dans au moins un des deux réservoirs (405, 410) en fonction d'une quantité de substance active, contenue dans la composition, à délivrer.

7. Dispositif (500) selon la revendication 3et l'une des revendications 1 à 6, dans lequel au moins un vaporisateur (520) est :
- un vaporisateur à coupelle échauffée par conduction ou convection ou
- un vaporisateur à résistance chauffante ou
- un vaporisateur à maillage chauffant.

8. Dispositif (700, 800) selon la revendication 3 et l'une des revendications 1 à 7, dans lequel la chambre intermédiaire (525) stocke un composé chauffé suite à la mise en actionnement d'au moins un vaporisateur (520) de composition active contenue dans un réservoir (505).

9. Dispositif (800) selon la revendication 8, dans lequel la chambre (525) intermédiaire comporte un élément (805) chauffant le composé stocké dans ladite chambre.

10. Dispositif (800) selon la revendication 9, dans lequel l'élément (805) chauffant est positionné à proximité d'un maillage ou d'une coupelle contenant le composé.

11. Dispositif (800) selon la revendication 10, dans lequel l'élément (805) chauffant est positionné au moins en partie autour et/ou au centre du composé.

12. Dispositif (700, 800) selon l'une des revendications 8 à 11, dans lequel la chambre (525) intermédiaire est amovible du dispositif.

## Patentansprüche

1. Tragbare Inhalationsvorrichtung (100, 200, 300, 400, 500, 600, 700, 800) wenigstens einer wirksamen Zusammensetzung, **dadurch gekennzeichnet, dass** sie umfasst:
- wenigstens zwei Speichertanks (105, 110, 205, 305, 405, 410, 505, 510, 605) einer wirksamen Zusammensetzung,
- wenigstens eine Zwischenkammer (115, 525), die an wenigstens einen Tank angeschlossen ist,
- eine Inhalationsleitung (120, 530), die mit der Zwischenkammer verbunden ist,
- wenigstens zwei Aerosolisierungsmittel (130, 135) und in dem wenigstens zwei Aerosolisierungsmittel (130, 135) jeweils einem eigenen Tank zugeordnet sind, und:
- wenigstens ein erstes Aerosolisierungsmittel (415) ein Entspannungsmittel eines mit Druck beaufschlagten Inhaltes ist und wenigstens ein zweites Aerosolisierungsmittel (420) ein Entspannungsmittel eines mit Druck beaufschlagten Inhaltes ist oder
- wenigstens ein erstes Aerosolisierungsmittel (230) ein Vernebler ist und wenigstens ein zweites Aerosolisierungsmittel (235) ein Zerstäuber ist oder
- wenigstens ein erstes Aerosolisierungsmittel (130) ein Vernebler ist und wenigstens ein zweites Aerosolisierungsmittel (306) ein Entspannungsmittel eines mit Druck beaufschlagten Inhaltes in einem Tank ist oder
- wenigstens ein erstes Aerosolisierungsmittel (520) ein Zerstäuber ist und wenigstens ein zweites Aerosolisierungsmittel (306) ein Entspannungsmittel eines mit Druck beaufschlagten Inhaltes in einem Tank ist,
- einen Regler (125, 225, 325, 425, 535) der Menge der wirksamen Zusammensetzung, die in Abhängigkeit von einem Befehl aerosoliert wird, der eine Menge der wirksamen Zusammensetzung darstellt, die vom Regler empfangen wird.

2. Vorrichtung (100, 200, 300) gemäß Anspruch 1, bei dem wenigstens ein Aerosolisierungsmittel ein Vernebler (130, 135, 230) der wirksamen flüssigen Zusammensetzung ist, die in einem Tank gespeichert ist und bei dem der Vernebler (130) umfasst:
- ein Sieb (140), das Öffnungen und ein Schwenkmittel (145) des Siebes aufweist, wobei der Regler (125) ein Verschlussmittel (150) wenigstens eines Teils der Öffnungen in Abhängigkeit von der Menge der zu aerosolierenden, wirksamen Zusammensetzung umfasst oder der Regler zum Steuern der Schwingbewegung des Siebes in einer Frequenz ausgestaltet ist, die in Abhängigkeit von der Menge und/oder den Merkmalen der zu vernebelnden wirksamen Zusammensetzung bestimmt ist, oder
- ein Mittel (160) für Schwingbewegungen einer Oberfläche, wobei der Regler (125) zum Steuern der Schwingbewegung der Oberfläche in einer Frequenz ausgestaltet ist, die in Abhängigkeit von der Menge der zu aerosolierenden wirksamen Zusammensetzung und/oder Merkmale der zu vernebelnden wirksamen Zusammensetzung bestimmt ist oder
- eine Vielzahl von Sieben (240, 250), die unterschiedliche Porositäten aufweisen, und ein Mittel (245) für unabhängige Schwingbewegungen jedes Siebes, wobei der Regler (225) zum Steuern der Schwingbewegung wenigstens eines der Siebe in Abhängigkeit von der Menge der zu aerosolisierenden wirksamen Zusammensetzung ausgestaltet ist.

3. Vorrichtung (200) gemäß einem der Ansprüche 1 bis 2, die einen Zerstäuber (235) mit einer wirksamen, flüssigen Zusammensetzung umfasst, die in einem Tank (205) gespeichert ist.

4. Vorrichtung (100) gemäß einem der Ansprüche 1 bis 3, bei der ein Tank eine wirksame Zusammensetzung umfasst, die eine wirksame Substanz enthält und der andere Tank eine wirksame Zusammensetzung umfasst; die eine geringere oder Null-Konzentration der genannten wirksamen Substanz umfasst, wobei jedes Aerosolierungsmittel, das einem genannten Tank zugeordnet ist, in Abhängigkeit von der Menge und/oder den Merkmalen der zu aerosolisierenden aktiven Zusammensetzung gesteuert ist.

5. Vorrichtung (300) gemäß einem der Ansprüche 1 bis 4, bei der wenigstens ein Tank (305) mit Druck beaufschlagt ist und Sauerstoff oder jedes andere, mit Druck beaufschlagte Gas umfasst, das eine wirksame Substanz in Form einer Lösung oder Suspension enthalten kann, mit der Zwischenkammer (115) verbunden ist, wobei die Aerosolisierung des Sauerstoffs oder des mit Druck beaufschlagten Gases von dem Regler (325) gesteuert ist, wobei wenigstens ein Tank an die Zwischenkammer (115) angeschlossen ist, die als Ausdehnungskammer für die darin freigesetzten Flüssigkeiten wirkt.

6. Vorrichtung (400) gemäß Anspruch 4, bei der der Regler (425) zum Steuern einer Aerosolisierungsdauer der wirksamen Zusammensetzung, die in wenigstens einem der zwei Tanks (405, 410) enthalten ist, in Abhängigkeit von einer auszugebenden Menge der wirksamen Substanz, die in der Zusammensetzung enthalten ist, ausgestaltet ist.

7. Vorrichtung (500) gemäß Anspruch 3 und einem der Ansprüche 1 bis 6, bei der wenigstens ein Zerstäuber (520):
- ein Zerstäuber mit per Leitung oder Konvektion erhitzter Kupelle oder
- ein Zerstäuber mit Heizwiderstand oder
- ein Zerstäuber mit Heizgitter ist.

8. Vorrichtung (700, 800) gemäß Anspruch 3 und einem der Ansprüche 1 bis 7, bei der die Zwischenkammer (525) eine Verbindung speichert, die nach der Betätigung wenigstens eines Zerstäubers (520) der in einem Tank (505) enthaltenen wirksamen Zusammensetzung erhitzt ist.

9. Vorrichtung (800) gemäß Anspruch 8, bei der die Zwischenkammer (525) ein Element (805) umfasst, das die in der genannten Kammer gespeicherte Zusammensetzung erhitzt.

10. Vorrichtung (800) gemäß Anspruch 9, bei der das erhitzende Element (805) in der Nähe eines Gitters oder einer Kupelle angeordnet ist, die die Zusammensetzung enthält.

11. Vorrichtung (800) gemäß Anspruch 10, bei der das erhitzende Element (805) wenigstens zum Teil um die und/oder im Zentrum der Verbindung angeordnet ist.

12. Vorrichtung (700, 800) gemäß einem der Ansprüche 8 bis 11, bei der die Zwischenkammer (525) von der Vorrichtung abnehmbar ist.

## Claims

1. Portable device (100, 200, 300, 400, 500, 600, 700, 800) for inhalation of at least one active composition, **characterized in that** it comprises:
- at least two reservoirs (105, 110, 205, 305, 405, 410, 505, 510, 605) for storing an active composition;
- at least one intermediate chamber (115, 525) connected to at least one reservoir;
- an inhalation line (120, 530) connected to the intermediate chamber;
- at least two aerosolization means (130, 135), and wherein at least two aerosolization means (130, 135) are each connected to a separate reservoir, and:
- at least a first aerosolization means (415) is a means for expanding a pressurized content and at least a second aerosolization means (420) is a means for expanding a pressurized content; or
- at least a first aerosolization means (230) is a nebulizer and at least a second aerosolization means (235) is a vaporizer; or
- at least a first aerosolization means (130) is a nebulizer and at least a second aerosolization means (306) is a means for expanding a pressurized content in a reservoir; or
- at least a first aerosolization means (520) is a vaporizer and at least a second aerosolization means (306) is a means for expanding a pressurized content in a reservoir;
- a regulator (125, 225, 325, 425, 535) for regulating the quantity of aerosolized active composition according to a command representing a quantity of active composition to be aerosolized received by the regulator.

2. Device (100, 200, 300) according to claim 1, wherein at least one aerosolization means is a nebulizer (130, 135, 230) for liquid active composition stored in a reservoir, and wherein the nebulizer (130) comprises:
- a screen (140) having openings, and a means (145) for making the screen oscillate, the regulator (125) comprising a means (150) for closing at least one portion of the openings according to the quantity of active composition to be aerosolized, or the regulator being configured to make the screen oscillate at a frequency determined as a function of the quantity and/or characteristics of the active composition to be nebulized; or
- a means (160) for making a surface oscillate, the regulator (125) being configured to make the surface oscillate at a frequency determined as a function of the quantity of active composition to be aerosolized and/or of characteristics of the active composition to be nebulized; or
- a plurality of screens (240, 250) having different porosities, and a means (245) for making each screen oscillate independently, the regulator (225) being configured to command the oscillation of at least one of the screens as a function of the quantity of the active composition to be aerosolized.

3. Device (200) according to one of claims 1 to 2, which comprises a vaporizer (235) for the active composition stored in the reservoir (205).

4. Device (100) according to one of claims 1 to 3, wherein one reservoir holds an active composition containing an active substance and the other reservoir holds an active composition containing a lower, or zero, concentration of said active substance, each aerosolization means associated with a said reservoir being controlled as a function of the quantity and/or characteristics of the active composition to be aerosolized.

5. Device (300) according to one of claims 1 to 4, wherein at least one reservoir (305) is pressurized, and holds oxygen or any other pressurized gas that can contain an active substance in the form of a solution or suspension, connected to the intermediate chamber (115), the aerosolization of the oxygen or pressurized gas being controlled by the regulator (325), at least one reservoir being connected to the intermediate chamber (115) acting as expansion chamber for the fluids being released there.

6. Device (400) according to claim 4, wherein the regulator (425) is configured to command a duration for aerosolizing the active composition contained in at least one of the two reservoirs (405, 410) as a function of a quantity of active substance, contained in the composition, to be delivered.

7. Device (500) according to claim 3 and one of claims 1 to 6, wherein at least one vaporizer (520) is:
- a cup vaporizer heated by conduction or convection; or
- a heating element vaporizer; or
- a heating mesh vaporizer.

8. Device (700, 800) according to claim 3 and one of claims 1 to 7, wherein the intermediate chamber (525) stores a heated compound following the activation of at least one vaporizer (520) for active composition contained in a reservoir (505).

9. Device (800) according to claim 8, wherein the intermediate chamber (525) comprises an element (805) heating the compound stored in said chamber.

10. Device (800) according to claim 9, wherein the heating element (805) is positioned near a mesh or a cup containing the compound.

11. Device (800) according to claim 10, wherein the heating element (805) is positioned at least in part around and/or in the center of the compound.

12. Device (700, 800) according to one of claims 8 to 11, wherein the intermediate chamber (525) can be removed from the device.
